# EUROPEAN PATENT APPLICATION

(11) **EP 3 970 850 A1**
(43) Date of publication of application: **23.03.2022**
(21) Application number: 21168783.5
(22) Date of filing: 16.04.2021
(51) Int. Cl.: B01J 21/06, B01J 23/66, B01J 35/00, B01J 35/02

(54) **SELF-DISINFECTING PHOTOCATALYST SHEET**

(30) Priority: 21.09.2020 US 202017027535
(71) Applicant: Aleddra Inc., Renton, WA 98057 (US)
(72) Inventor: MAA, Chia-Yiu, Bellevue, 98006 (US); YU, Chun-Te, Bellevue, 98008 (US)
(74) Representative: Klunker IP Patentanwälte PartG mbB

(57) **Abstract**

A self-disinfecting photocatalyst sheet includes a substrate material and a photocatalyst layer with a primary photocatalyst and a secondary photocatalyst. The primary photocatalyst is a metal oxide photocatalyst, whereas the secondary photocatalyst is a metallic photocatalyst. The primary photocatalyst forms a covalent bond with the substrate material. The self-disinfecting photocatalyst sheet is activatable by a visible light and can self-disinfect against bacteria and viruses. A method of manufacturing the self-disinfecting photocatalyst sheet and means for alerting the user on the expiration of the self-disinfecting photocatalyst sheet are also presented.

## Description

### BACKGROUND

### TECHNICAL FIELD

The present disclosure pertains to the field of antimicrobial photocatalyst device and, more specifically, proposes a self-disinfecting photocatalyst sheet.

### DESCRIPTION OF RELATED ART

The COVID-19 pandemic has triggered a strong demand for disinfecting facilities, including both air and surface disinfection. For air disinfection, there are sustainable solutions such as upper-room germicidal UV equipment or air-disinfecting photocatalytic device disclosed in US Patent Application US 16/995,588 that could continually disinfect the air without any health concern. However, there are no solutions for providing a continual surface disinfection. Currently, there are two general approaches for surface disinfection. The first approach is to spray chemical disinfectant to a surface, and the second approach is to shine a UVC light with a central wavelength at 254nm to a surface. Both approaches are effective for surface disinfection. However, they could only be used when no users in the space because there are side effects or health concerns when exposing people directly to chemical disinfectant (causing skin or respiratory allergy) or UVC light (causing skin and eye damages). As a result, chemical disinfectant and UVC equipment could only be applied periodically during off hours. In-between the applications of chemical disinfectant and UVC equipment, bacteria and viruses will grow on a surface due to physical contacts to the surface by users or through the landing of airborne droplets emitted from human carriers onto the surface. A better surface disinfection solution would need to be an approach that could disinfect the surface continually and without introducing any side effect to users.

Photocatalysts are known to become active under ultraviolet light and kill bacteria by breaking down the cell wall of the bacteria. Soma, R., et al., in U.S. Pat. No. 6,242,752 teaches the use of a photocatalytic film made of anatase-type titanium dioxide (TiO₂) on the lens of a lighting device such that, as the light originating from the lighting device shines through the titanium oxide film, the UV rays of the light activate the photocatalyst, causing it to break down the bacteria cell wall and resulting in the killing of the bacteria. It is then feasible to create a photocatalyst sheet with a base material and a photocatalyst layer based on Soma's teaching. However, such photocatalyst sheet is photocatalytic responsive only to UV light source, but UV light source should not be used with users nearby. Therefore, such photocatalyst sheet fails to provide a continual surface disinfection.

In U.S. Pat. No. 9,522,384, Liu L. et al. teaches the use of rhombus-shape anatase-type titanium dioxide (TiO₂) such that this new type of TiO₂ can be activated by visible light wavelengths and become germicidal active. When making a photocatalyst sheet with this new type of TiO₂ introduced by Liu, such photocatalyst sheet would be photocatalytic responsive to a regular indoor lighting source with visible light wavelengths. This photocatalyst sheet may thus be used at all time and provide continual surface disinfection. There are however new issues with this visible-light responsive photocatalyst sheet. Firstly, A direct aerosol deposition of the new type of TiO₂ onto a base material may not provide strong enough bonding between this TiO₂ photocatalyst layer and the base material. As a result, the TiO₂ photocatalyst layer may not withstand the forces of normal use and could be peeled off the base material quickly. The continual surface disinfection of this photocatalyst sheet would be short lived. It is necessary to devise a way to bond the photocatalyst layer firmly to the base layer that could withstand the normal wear-and-tear. Secondly, even there is a strong bonding between the photocatalyst layer and the base layer, the photocatalyst layer could still be worn off due the wear-and-tear of normal use. When that happened, the photocatalytic disinfection would become less effective. Not knowing the photocatalyst layer being worn off, the user would use the photocatalyst sheet under a false sense of surface disinfection protection, which could be even worse than not having any surface disinfection protection.

The present disclosure proposes a self-disinfecting photocatalyst sheet that can provide a continual surface disinfection against bacteria and viruses, while solving the issues raised above and ensuring an effective surface disinfection.

### SUMMARY

In one aspect, the self-disinfecting photocatalyst sheet comprises a substrate material and a photocatalyst layer. The substrate material has a first side and a second side. The photocatalyst layer contains a primary photocatalyst and a secondary photocatalyst. The primary photocatalyst is a metal oxide photocatalyst, whereas the secondary photocatalyst is a metallic photocatalyst. The mass ratio of the primary photocatalyst to the secondary photocatalyst is 10:1 to 100:1. With the mass ratio, the metallic photocatalyst is appropriately categorized as a secondary photocatalyst, and should not be considered as a co-photocatalyst to the primary photocatalyst, for it does not contribute the photocatalytic activities at the same level as that of the primary photocatalyst.

One novel feature of the self-disinfecting photocatalyst sheet is that the primary photocatalyst forms a covalent bond with the substrate material at the molecular level on the first side of the substrate material. The covalent bond is a type of chemical bond. Unlike a physical bond (such as using adhesive) where two materials bonded together are still distinguishable and separable by force, the two materials involved in the covalent bond are intertwined at the molecular level and are thus inseparable. The covalent bond between the primary photocatalyst layer and the substrate material is stronger than a physical bond and can withstand the forces of normal use. As a result, the photocatalyst layer of the disclosure would not be peeled off the substrate material. The metal oxide photocatalyst is chosen as the primary photocatalyst of the present disclosure because it can form a covalent bond with the substrate material. The secondary photocatalyst which is a metallic photocatalyst cannot form a valent boding with the substrate material. Optionally, an adhesive layer is coated on the second side of the substrate material such that the present disclosure may adhere to a surface for providing self-disinfection protection for that surface.

In some embodiments, the primary metal oxide photocatalyst includes anatase titanium dioxide (TiO₂) because the anatase-type titanium (IV) dioxide (TiO₂) is known to have the best photocatalytic property among TiO₂ material family. In some embodiments, the primary photocatalyst comprises rhombus-shape anatase titanium dioxide (TiO₂) and has a major axis 10-15 nm and a minor axis 3-6 nm. This rhombus-shape anatase TiO₂ has a much higher volume density than that of the standard spherical-shape anatase TiO₂. Therefore, when the rhombus-shape anatase TiO₂ is activated, it could generate a lot more activated photocatalytic particles than that of the spherical-shape anatase TiO₂, resulting a more effective disinfection against pathogens.

In some embodiments, the secondary metallic photocatalyst may include silver, gold, copper, zinc, nickel, cerium, or a combination thereof. It is foreseeable to use other metal elements as the secondary photocatalyst. The metallic photocatalyst not only contributes to the photocatalytic activity itself, but also enables the primary metal oxide photocatalyst to absorb spectral energy in the visible light wavelength and become photocatalytic active. In some embodiments, the photocatalyst layer is activatable by a visible light in a wavelength range greater than 400nm. With this feature, the self-disinfecting photocatalyst sheet when used in an indoor environment can thus be photocatalytic active with a visible light emitted from an indoor light source and can self-disinfect its surface continually against the microbial.

The secondary metallic photocatalyst may contain one or two or even more metallic photocatalyst materials. In some embodiments, the secondary photocatalyst only comprises two metallic photocatalyst materials, a third metallic photocatalyst and a fourth metallic photocatalyst. In some embodiments, the third metallic photocatalyst comprises silver nanoparticles (NPs) and the fourth metallic photocatalyst comprises cerium NPs. Both silver NPs and cerium NPs help improve the photocatalytic activity of anatase TiO₂ when illuminated with a visible light. It is found that silver NPs themselves are effective in inhibiting bacteria under a visible light, whereas cerium NPs are effective in inhibiting viruses under a visible light. Having both silver NPs and cerium NPs as the secondary metallic photocatalyst would improve the self-disinfection effectiveness of the present disclosure under a visible light.

In some embodiments, the substrate material comprises a glass. Some of the cellphone screen protectors are made of soda lime glass or alkaline-aluminosilicate glass, and they can be made to be a very thin sheet. They would be good candidates for the substrate material of the present disclosure. A screen protector with a self-disinfecting photocatalytic surface provides the user of the cellphone a continual antimicrobial protection against any germs on the screen protector.

In some embodiments, the substrate material comprises a resin. The resin has been widely used for screen protector, packaging, and surface covering. Some widely used resin includes polyvinyl chloride, polyethylene, polyethylene terephthalate, polyurethane, thermoplastic polyurethane, polypropylene, polystyrene, silicone, and other thermoplastic and thermosetting resins. Once a resin forms a covalent band with the proposed primary photocatalyst, the surface of the resin product would become antimicrobial and self-disinfecting. Such resin could then be used for covering any surface, flat or not, and providing a continual disinfection protection for the surface.

In some embodiments, the optional adhesive layer comprises a pressure-sensitive adhesive (PSA) material. PSAs, especially acrylic based PSAs, may be tailored to provide a number of desirable attributes such as elasticity, tackiness, transparency, resistance to oxidation and sunlight, as well as have the necessary degree of adhesion and cohesion for different applications. With a PSA layer, the present disclosure may be attached, removed, and reattached to a surface, and provide a self-disinfection protection for the surface.

In some other embodiments, the optional adhesive layer comprises an electrostatic-enhancing agent for the purpose of adhering to a surface made of a material capable of being adhered to via electrostatic. Plastic, ceramic, and glass are materials that can be adhered to via electrostatic. The electrostatic-enhancing agent may be epoxy resin, polyepoxide, or graphene. It is foreseeable that the substrate material may contain an electrostatic-enhancing agent such as graphene, and in such case, it would not be necessary to add an electrostatic-enhancing agent layer to the substrate material.

The covalent bond between the primary photocatalyst and the substrate material is strong, so the photocatalyst layer would not be peeled off the substrate material. However, the photocatalyst layer may still be worn off due to the normal wear-and-tear. It would be necessary to alert a user when the photocatalyst layer is being worn off or when the self-disinfection function of the photocatalyst layer is about to expire. In some embodiments, the present disclosure also includes a color-changing dye that is coated on at least a portion of the photocatalyst layer for alerting the user the expiration of the self-disinfecting photocatalyst sheet through the change of its color. In some embodiments, the color-changing dye includes a phenol red. A phenol red is red when detecting not bacteria. It would change its color to orange when detecting bacteria. After coating a phenol red on the photocatalyst layer, the phenol red would thus remain red if the photocatalyst layer is still photocatalytic effective and keeps its surface bacteria free. When the photocatalyst layer become ineffective in disinfecting against bacteria, the phenol red would turn its color to orange. By observing the color of the phenol red coating on the present disclosure, a user would be able to tell whether it is time to replace the self-disinfecting photocatalyst sheet. In some other embodiments, the color-changing dye includes silver nanoparticles (NPs). Silver NPs has a brown color when first exposed to the air. It would gradually turn to a purple color due to oxidation. By controlling the amount of silver NPs, its color changing feature can thus be used as a timer. When it becomes dark purple, a user could take the visual que and replace the self-disinfecting photocatalyst sheet.

The photocatalytic property of the photocatalyst layer would drop when the photocatalyst layer of the present disclosure is worn off due to the normal wear-and-tear. It would be reasonable to use a dye that could also be worn off or faded away due to the normal wear-and-tear, and the wearing-off or fading-away of the dye could serve as an indicator of the wear-and-tear of the photocatalyst layer. Therefore, by observing the wearing-off or fading-away of the dye on the self-disinfecting photocatalyst sheet, a user can tell whether the sheet is due for replacement. In some embodiments, the sheet also includes a dye coated on at least a portion of the photocatalyst layer wherein the wearing-off or fading-away of the dye is a proxy to the wear-and-tear of the photocatalyst layer.

In some embodiments, the present disclosure is transparent and has a transmission rate of visible light greater than 90%. It is foreseeable that additional material(s) may be added to the substrate material or additional coating(s) may be added on the second side of the substrate material for different applications, such as filtering out UV light or blue light, or adding tint to reduce the spectral penetration rate of a visible light.

A method for producing the present disclosure includes the following steps. Step 1 is a surface activation step. In Step 1, a low-temperature atmospheric plasma is applied to the first side of the substrate material for improving the hydrophilic property of the substrate material. A substrate material such as glass or resin may have a low hydrophilic property by nature. It is difficult to coat water-based solution to the surface of glass or resin. By applying a low-temperature atmospheric plasma to the substrate material, the hydrophilic property of the substrate material can be greatly improved for a short time for the subsequent coating with a water-based solution. The low-temperature atmospheric plasma may be an oxygen plasma or a nitrogen plasma. The time and the temperature of the atmospheric plasma application would depend on the substrate material. For example, the time is shorter and the temperature is lower when using an atmospheric plasma to surface-activate a resin substrate material, whereas the time is longer and the temperature is higher when using the atmospheric plasma to surface-activate a glass substrate material. Step 2 is a surface coating step. In Step 2, a water-based photocatalyst solution is sprayed on the first side of the substrate material evenly. The water-based solution comprises greater than 98wt% in water and less than 2wt% in the primary photocatalyst and secondary photocatalyst. The mass ratio of the primary photocatalyst to the secondary photocatalyst is 10:1 to 100:1. Step 3 is a curing step. A heat curing is applied to the substrate material sprayed with the water-based photocatalyst solution. The heat creates a hydroxyl group out of the primary photocatalyst suitable for forming a covalent bond with the substrate material on the first side of the substrate material. The higher the temperature and the longer the heat curing process, the more primary photocatalyst molecules would generate a hydroxyl group, resulting in covalent bond between more primary photocatalyst molecules and more substrate material molecules on the first side of the substrate material. Step 4 is an optional step for coating an adhesive layer on the second side of the substrate material. The adhesive layer may comprise a pressure-sensitive adhesive (PSA) material or an electrostatic-enhancing agent.

### BRIEF DESCRIPTION OF THE DRAWINGS

The accompanying drawings are included to aid further understanding of the present disclosure and are incorporated in and constitute a part of the present disclosure. The drawings illustrate a select number of embodiments of the present disclosure and, together with the detailed description below, serve to explain the principles of the present disclosure. It is appreciable that the drawings are not necessarily to scale, as some components may be shown to be out of proportion to size in actual implementation in order to clearly illustrate the concept of the present disclosure.
FIG. 1 schematically depicts a diagram of an embodiment of the present disclosure with adhesive coating on the second side of a substrate material.
FIG. 2 schematically depicts a diagram of another embodiment of the present disclosure coated with a dye on the photocatalyst layer.

### DETAILED DESCRIPTION OF PREFERRED EMBODIMENTS

### OVERVIEW

Various implementations of the present disclosure and related inventive concepts are described below. It should be acknowledged, however, that the present disclosure is not limited to any particular manner of implementation, and that the various embodiments discussed explicitly herein are primarily for purposes of illustration. For example, the various concepts discussed herein may be suitably implemented in a variety of the primary, the secondary photocatalyst, the substrate material, and the dye.

The present disclosure discloses a self-disinfecting photocatalyst sheet includes a substrate material and a photocatalyst layer with a primary photocatalyst and a secondary photocatalyst. The primary photocatalyst is a metal oxide photocatalyst, whereas the secondary photocatalyst is a metallic photocatalyst. The primary photocatalyst forms a covalent bond with the substrate material. The self-disinfecting photocatalyst sheet is activatable by a visible light and can self-disinfect against bacteria and viruses.

### EXAMPLE IMPLEMENTATIONS

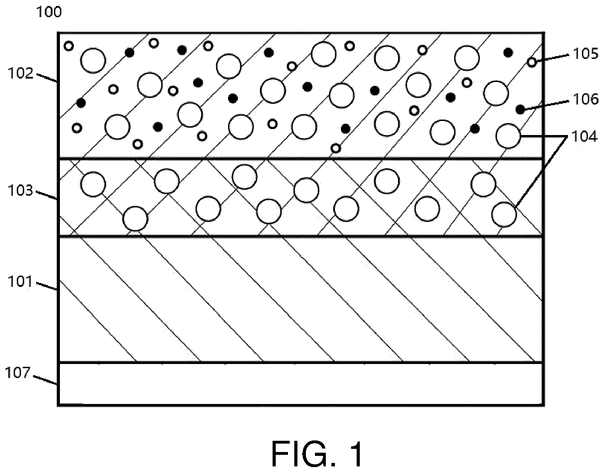

In FIG. 1, an embodiment 100 of the present disclosure is shown. A photocatalyst layer 102 is coated over a substrate material, polyvinyl chloride (PVC) 101. The photocatalyst layer contains a primary metal oxide photocatalyst TiO₂ 104 and two secondary metallic photocatalysts, silver nanoparticles (NPs) 105 and cerium NPs 106. The primary photocatalyst TiO₂ 104 forms a covalent bond with the PVC substrate material 101 along the boundary 103 where the photocatalyst layer 102 meets the substrate material 101. With the presence of the two secondary photocatalysts, the photocatalyst layer 102 is activatable by a visible light with a wavelength range greater than 400nm. The second side of the PVC substrate material is coated with an adhesive layer 107 comprising a pressure-sensitive adhesive (PSA) material. With a PSA layer, the embodiment could be used as self-disinfecting window film, and it can be attached, removed, and even reattached to a glass window and provide self-disinfection protection for the glass window.

This embodiment is made firstly by treating the PVC substrate material 101 with an oxygen plasma at 50 °C. Then one side of the plasma-treated substrate material PVC is sprayed with a water-based TiO₂ solution that also contains silver NPs and cerium NPs. The TiO₂, silver NPs, and cerium NPs, together has less than 2wt% of the solution, and the water has 98wt% of the solution. The mass ratio of the secondary photocatalysts, silver NPs and cerium NPs, to the primary photocatalyst TiO₂ is 30:1. Thirdly, the substrate material being sprayed with a water-based TiO₂ solution goes through a heat curing process for 30 minutes at 150 °C. Lastly, the second side of the PVC substrate material is coated with an adhesive layer comprising a pressure-sensitive adhesive (PSA) material.

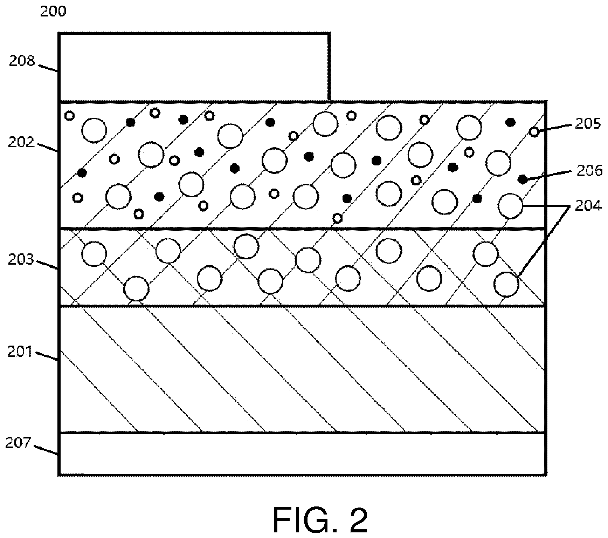

In FIG. 2, another embodiment 200 of the present disclosure is shown. Its substrate material, soda lime glass 201, is coated with a photocatalyst layer 202, comprising a primary metal oxide photocatalyst TiO₂ 204, and two secondary metallic photocatalysts, silver NPs 205 and cerium NPs 206. The primary photocatalyst TiO₂ 204 forms a covalent bond with the substrate material 201 along the boundary 203 where the photocatalyst layer 202 meets the substrate material 201. A layer of phenol red 208 is coated over a portion of the photocatalyst layer 202. The phenol red layer is red when this embodiment is first used under a visible light, since the photocatalyst layer 202 is activatable by the visible light and self-disinfect against bacteria, and therefore the phenol red layer detects no bacteria. As the photocatalyst layer 202 is worn off due the normal wear-and-tear, its photocatalytic effectiveness begins to drop and leaves some bacteria on its surface. When enough bacteria are accumulated on the surface of the photocatalyst layer 202, they will be detected by the phenol red layer 208, causing the phenol red to change its color to orange. By observing the color changing of the phenol red layer 208, a user can decide when it is necessary to replacement the self-disinfecting photocatalyst sheet with a new one.

The second side of the soda lime glass substrate material is coated with an adhesive layer comprising an electrostatic-enhancing agent 207 and this embodiment 200 has a transmission rate of visible light greater than 90%. This embodiment could be used as a cellphone screen protector for it could be adhered to the cellphone screen via the electrostatic-enhancing layer 207 without blocking the content of the cellphone screen, and more importantly, provide a self-disinfection protection for the cellphone screen.

### ADDITIONAL AND ALTERNATIVE IMPLEMENTATION NOTES

Although the techniques have been described in language specific to certain applications, it is to be understood that the appended claims are not necessarily limited to the specific features or applications described herein. Rather, the specific features and examples are disclosed as non-limiting exemplary forms of implementing such techniques.

As used in this application, the term "or" is intended to mean an inclusive "or" rather than an exclusive "or." That is, unless specified otherwise or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims should generally be construed to mean "one or more," unless specified otherwise or clear from context to be directed to a singular form.

## Claims

1. A self-disinfecting photocatalyst sheet, comprising
a substrate material with a first side and a second side opposite the first side;
and
a photocatalyst layer comprising a primary photocatalyst and a secondary photocatalyst,
wherein:
the primary photocatalyst comprises a metal oxide photocatalyst,
the secondary photocatalyst comprises a metallic photocatalyst,
a mass ratio of the primary photocatalyst to the secondary photocatalyst is between 10:1 to 100:1, and
the primary photocatalyst forms a covalent bond with the substrate material at a molecular level on the first side of the substrate material.

2. The self-disinfecting photocatalyst sheet of claim 1, wherein an adhesive layer is coated on the second side of the substrate material.

3. The self-disinfecting photocatalyst sheet of claim 1, wherein the primary photocatalyst further comprises anatase titanium dioxide (TiO₂).

4. The self-disinfecting photocatalyst sheet of claim 3, wherein the primary photocatalyst further comprises rhombus-shape anatase titanium dioxide (TiO₂) and has a major axis of 10-15 nm and minor axis of 3-6 nm.

5. The self-disinfecting photocatalyst sheet of claim 1, wherein the secondary photocatalyst further comprises silver, gold, copper, zinc, nickel, cerium, or a combination thereof.

6. The self-disinfecting photocatalyst sheet of claim 5, wherein the secondary photocatalyst further comprises a third metallic photocatalyst and a fourth metallic photocatalyst with no other metallic photocatalysts.

7. The self-disinfecting photocatalyst sheet of claim 6, wherein the third metallic photocatalyst comprises silver nanoparticles (NPs) and the fourth metallic photocatalyst comprises cerium NPs.

8. The self-disinfecting photocatalyst sheet of claim 1, wherein the photocatalyst layer is activatable by a visible light in a wavelength range greater than 400nm.

9. The self-disinfecting photocatalyst sheet of claim 1, wherein the substrate material comprises a glass.

10. The self-disinfecting photocatalyst sheet of claim 1, wherein the substrate material comprises a resin.

11. The self-disinfecting photocatalyst sheet of claim 2, wherein the adhesive layer comprises a pressure-sensitive adhesive (PSA) material.

12. The self-disinfecting photocatalyst sheet of claim 2, wherein the adhesive layer comprises an electrostatic-enhancing agent.

13. The self-disinfecting photocatalyst sheet of claim 1, further comprising:
a color-changing dye coated on at least a portion of the photocatalyst layer, the color-changing dye configured to alert a user an expiration of the self-disinfecting photocatalyst sheet through a change of a color of the color-changing dye.

14. The self-disinfecting photocatalyst sheet of claim 13, wherein the color-changing dye comprises a phenol red.

15. The self-disinfecting photocatalyst sheet of claim 13, wherein the color-changing dye comprises silver nanoparticles (NPs).

16. The self-disinfecting photocatalyst sheet of claim 1, further comprising:
a dye coated on at least a portion of the photocatalyst layer,
wherein wearing-off or fading-away of the dye is a proxy to wear-and-tear of the photocatalyst layer.

17. The self-disinfecting photocatalyst sheet of claim 1, wherein the sheet has a transmission rate of visible light greater than 90%.

18. A method for producing a self-disinfecting photocatalyst sheet, comprising:
forming the self-disinfecting photocatalyst sheet having a substrate material with a first side and a second side opposite the first side and a photocatalyst layer, with the photocatalyst layer comprising a primary photocatalyst and a secondary photocatalyst, by performing:
applying a low-temperature air atmospheric plasma to the first side of the substrate material to improve a hydrophilic property of the substrate material;
spraying a water-based photocatalyst solution on the first side of the substrate material, wherein the water-based solution comprises greater than 98wt% in water, less than 2wt% in the primary photocatalyst and secondary photocatalyst, and a mass ratio of the primary photocatalyst to the secondary photocatalyst is between 10:1 to 100:1;
performing a heat curing process to create a hydroxyl group out of the primary photocatalyst to form a covalent bond with the substrate material on the first side of the substrate material; and
coating an adhesive layer on the second side of the substrate material.
